# EUROPEAN PATENT SPECIFICATION

(11) **EP 3 883 530 B1**
(45) Date of publication and mention of the grant of the patent: **16.04.2025**
(21) Application number: 19842641.3
(22) Date of filing: 17.12.2019
(51) Int. Cl.: A61K 8/19, A61K 8/21, A61K 8/24, A61K 8/27, A61Q 11/00, A61P 1/02, A61P 29/00

(54) **METHODS OF INHIBITING NEUTROPHIL RECRUITMENT TO THE GINGIVAL CREVICE**
VERFAHREN ZUR HEMMUNG DER NEUTROPHILENREKRUTIERUNG IN DEN GINGIVASULCUS
PROCÉDÉS D'INHIBITION DU RECRUTEMENT DES NEUTROPHILES AU NIVEAU DU SULCUS GINGIVAL

(30) Priority: 26.12.2018 US 201862785132 P
(43) Date of publication of application: 29.09.2021
(73) Proprietor: Colgate-Palmolive Company, New York, NY 10022 (US)
(72) Inventor: ZENOBIA, Camille, Hampton, NJ 08827 (US); DAEP, Carlo, Avenel 07001, New Jersey (US); CHEN, Dandan, Bridgewater, NJ 08807 (US); TRIVEDI, Harsh Mahendra, Hillsborough, New Jersey 08844 (US); MASTERS, James, Ringoes, NJ 08551 (US)
(74) Representative: Sonnenhauser, Thomas Martin
(86) International application number: PCT/US2019/066866
(87) International publication number: WO 2020/139628

(56) References cited:
- WO-A1-2018/005335
- US-A1- 2007 053 849
- US-A1- 2017 367 946
- US-A1- 2017 367 947
- US-A1- 2017 367 953
- ANONYMOUS: "Scientific Evidence Behind. ADVANCED DENTIFRICE TECHNOLOGIES", 20070101, 1 January 2007 (2007-01-01), pages 1 - 164, XP007920425
- J. KIM ET AL: "Anti-inflammatory effects of zinc in PMA-treated human gingival fibroblast cells", MEDICINA ORAL PATOLOGÍA ORAL Y CIRUGIA BUCAL, 1 January 2015 (2015-01-01), pages e180 - e187, XP055672771, DOI: 10.4317/medoral.19896
- CHEN ZETAO ET AL: "Osteoimmunomodulation for the development of advanced bone biomaterials", MATERIALS TODAY, ELSEVIER, AMSTERDAM, NL, vol. 19, no. 6, 11 December 2015 (2015-12-11), pages 304 - 321, XP029663387, ISSN: 1369-7021, DOI: 10.1016/J.MATTOD.2015.11.004
- O'NEILL EDWARD ET AL: "The roles of ions on bone regeneration", DRUG DISCOVERY TODAY, ELSEVIER, AMSTERDAM, NL, vol. 23, no. 4, 3 February 2018 (2018-02-03), pages 879 - 890, XP085369780, ISSN: 1359-6446, DOI: 10.1016/J.DRUDIS.2018.01.049

## Description

### BACKGROUND

The gums, also referred to as gingiva, are a part of the soft tissue lining of the mouth that surround the teeth and provide a seal around them. The gingival margin is the interface between the sulcular epithelium and the epithelium of the oral cavity. This interface exists at the most coronal point of the gingiva, otherwise known as the crest of the marginal gingiva. The gingival crevice, also called gingival sulcus, is the space located around a tooth between the wall of the unattached gum tissue and the enamel and/or cementum of the tooth.

Gum disease is an inflammation of the gum line that can progress to affect the bone that surrounds and supports your teeth. The three stages of gum disease are, from least to most severe, gingivitis, periodontitis and advanced periodontitis.

Gingivitis is the initial stage of gum disease. The direct cause of gingivitis is plaque - the soft, sticky, colorless film of bacteria that forms constantly on the teeth and gums. If the plaque is not removed by daily brushing and flossing, accumulates and hardens over time. The bacteria found in this buildup produces toxins that can irritate the gum tissue, causing gingivitis.

Gingivitis is treatable. Damage can be reversed, since the bone and connective tissue that hold the teeth in place are not yet affected. Left untreated, however, gingivitis can become an advanced stage of gum disease, periodontitis, and cause permanent damage to teeth and jaw.

The bacteria produced by the plaque irritate the gums, triggering the immune system to produce powerful bacteria-fighting elements that attack the infection. An unfortunate consequence is that these elements inadvertently destroy bone and tissue responsible for supporting the teeth. Essentially the body turns on itself. As tissue is broken down, spaces begin to form separating the gums from the teeth. These spaces become infected and deepen, further destroying gum tissue and bone. Eventually when there is an insufficient amount of bone left to support teeth, they begin to feel loose and may have to be removed.

Neutrophils, which are a type of granulocyte, are an abundant type of white blood cells that form an essential part of the innate immune system. During the beginning phase of inflammation in the gums, neutrophils are one of the first-responders of inflammatory cells to migrate towards the site of inflammation. The migration of neutrophils is regulated by various chemical signals in a process called chemotaxis.

Examples of proteins that can reduce neutrophil recruitment include IL-1Ra and sICAM, and examples of proteins that induce neutrophil recruitment are selected from the group consisting of: TNF-a, RANTES, MIF, GCSF, CXCL10 and GRO. Increasing levels of proteins that reduce neutrophil recruitment such as IL-1Ra and sICAM in the gingival crevice have an anti-inflammatory effect that reduces the negative consequences of inflammation of the gums. Likewise, reducing levels of proteins that promote neutrophil recruitment such as TNF-a, RANTES, MIF, GCSF, CXCL10 and GRO in the gingival crevice have an anti-inflammatory effect that reduces the negative consequences of inflammation of the gums.

Reducing inflammation of the gums by reducing neutrophil recruitment in the gingival crevice can resolve or reduce the severity of gingivitis and have a role in treatment of advanced gum disease such as periodontitis and advanced periodontitis.

US 2017/367946 A1 discloses an oral care composition, comprising: an orally acceptable carrier; zinc phosphate; and stannous fluoride. US 2017/367947 A1 discloses a high water oral care composition comprising an orally acceptable carrier, zinc phosphate, stannous fluoride, and an organic acid buffer system.

### BRIEF SUMMARY

Oral care compositions as defined in the claims comprising: zinc phosphate, stannous fluoride and optionally, an organic acid buffer system for use in a method of increasing proteins that reduce signals associated with neutrophil recruitment and reducing proteins that induce neutrophil recruitment within an individual's gingival crevice, wherein the proteins that induce neutrophil recruitment are TNFα and MIF, are provided. The method comprises applying to the individual's oral cavity in an amount effective to increase proteins that reduce signals associated with neutrophil recruitment and that reduce proteins that induce neutrophil recruitment within an individual's gingival crevice.

### BRIEF DESCRIPTION OF THE DRAWINGS

Figure 1 is an illustration of a GCM.
Figure 2 shows data from GCM experiments in Example 1 comparing TNFa levels in response to treatment with a toothpaste formulation.
Figure 3 shows data from GCM experiments in Example 1 comparing MIF levels in response to treatment with a toothpaste formulation.

### DETAILED DESCRIPTION

Application of oral care compositions to the oral cavity of an individual can affect the inflammatory signals in the individual's gingival crevice including the reduction of pro-inflammatory signals and the upregulation of signals that block or reduce inflammation. Reducing signals which recruit neutrophils and promoting signals that reduce neutrophil recruitment provides effective strategies to control inflammation and promote healthy gums and overall good oral health.

A model has been designed for testing biological efficacy of oral health compounds. The model employs a unique combination of cells and bacterial biofilm in an *in vitro* cell culture that allows for the measure of inflammatory biomarkers that are predictive of clinical effects. The model is helpful in predicting product efficacy.

The model, which is referred to as a gingival crevice model (GCM), includes layered primary gingival epithelial cells, such as tissue commercially available from MatTek), coupled with neutrophil-like cells that are generated by inducing HL60 cells (ATCC) to a neutrophil like phenotype with retinoic acid. The model simulates what is seen morphologically within healthy junctional gingival tissues. An *ex vivo* derived biofilm, generated from saliva donation and created on substrates, such as HAP disks, poly-D-lysine-coated substrates, collagen-coated substrates, enamel disks, collagen matrices, and polydimethylsiloxane (PDMS), agarose, agar, poly(ethylene glycol) dimethacrylate (PEGDMA) and 2-methacryloyloxyethyl phosphorylcholine polymer (PMPC) hydrogels, is added to the epithelial cell layer. To simulate an inflammatory disease-like state within the model system, Fetal Bovine serum may be added. The model allows for rapid analysis of oral care products such as toothpaste, mouthwash, etc.

The GCM is useful to test a compound or formulation's ability to prevent or resolve inflammation. The GCM is also useful to test the compound or formulation's effect on oral bacteria and biofilm, which are generated by saliva donation and cultivation on substrates, such as HAP, poly-D-lysine, collagen-coated, enamel disks or on "soft" substrates such as, for example, substrates made from collagen matrices such as CollaForm^{®} Collagen Wound Dressing material (Impladent Ltd., Jamaica, NY), or substrates made from polydimethylsiloxane (PDMS), agarose, agar, poly(ethylene glycol) dimethacrylate (PEGDMA), and 2-methacryloyloxyethyl phosphorylcholine polymer (PMPC) hydrogels, to predict health or disease status. The model provides predicative clinical measures.

Using the GCM, formulations were tested and analyzed for effects in chemokine/cytokine production. An oral care composition that comprises an orally acceptable carrier, zinc phosphate and stannous fluoride was found to effect signals associated with neutrophil recruitment and a promote signals that reduce neutrophil recruitment. Oral care composition that reduce signals associated with neutrophil recruitment and promote signals that reduce neutrophil recruitment are useful to control inflammation and to promote healthy gums and overall good oral health.

In some embodiments, the zinc phosphate is a preformed salt of zinc phosphate. "Preformed salt" when used in reference to zinc phosphate means that the zinc phosphate is not formed *in situ* in the oral care composition, e.g., through the reaction of phosphoric acid and another zinc salt. The zinc phosphate may be present in an amount sufficient so that the stannous fluoride dissociates to provide a therapeutically effective amount of stannous ions in aqueous solution. The amount of zinc phosphate is preferably from 0.05 to 10% by weight relative to the weight of the oral care composition, preferably from 0.05 to 5% by weight, relative to the weight of the oral care composition, for example, for example, from 0.1 to 8% by weight or from 0.1 to 4% by weight, or from 0.5 to 5% by weight, or from 0.5 to 4% by weight, or from 0.5 to 3% by weight, or from 0.5 to 2% by weight, or from 0.8 to 1.5% by weight, or from 0.9 to 1.1% by weight, or about 1% by weight, or from 1 to 4%, or from 1 to 3% by weight, or from 2 to 3% by weight, or about 2%, or about 2.25% or about 2.5%, by weight.

The amount of the stannous fluoride is preferably from 0.01% to 11% by weight from 0.01% to 5% by weight, relative to the weight of the oral care composition, for example, from 0.05 to 4% by weight, or from 0.1% to 3% by weight, from 0.05% to 11% by weight, relative to the weight of the oral care composition, for example, from 0.05 to 7% by weight, or from 0.1% to 5% by weight, or from 0.2 to 3% by weight, or from 0.2 to 2% by weight, or from 0.2 to 1% by weight, or from 0.2 to 0.8% by weight, or from 0.3 to 1% by weight, or from 0.4 to 0.8% by weight, or from 0.4 to 0.6% by weight, or from 0.4 to 0.5% by weight, or about 0,45%> by weight (e.g., 0.454%).

In some embodiments, the amount of the water is 10% by weight or more, or about 12% by weight or more, relative to the weight of the oral care composition, for example, 10-90%, or 10-80%, or 10-70%, or 10-60%, or 10-50%, or 10-40%, or 10-30%, or from 15% to 85%, or 15-30%,or from 20% to 75%, or from 20-50% or from 20% to 40% or from 20% to 30%, or from 25% to 50%, or from 30% to 40%, or 30-35%, for example, about 35%, or about 30%, or about 25% or about 20%. In some embodiments, toothpastes and oral gels may comprise from 1.0% to 99% water, by weight of the composition. For example, the composition may comprise at least 10%, 15%, 20%, 25%, 30%, 35% or 40% water, up to a maximum of, for example, 60%, 70%, 80%, 90%, 95% or 99% water, by weight of the composition. As used herein, amounts of water refer to water added directly to the composition, as well as water added as part of ingredients or components which are added as aqueous solutions. In some embodiments, the composition comprises 10-60% water, or 10-50% water, or 10-40% water, or 10-30% water, or 15-30% water, or 20-30% water, or about 25% water, by weight of the composition.

The optional organic buffer system may comprise a carboxylic acid and one or more conjugate base salts thereof, for example, alkali metal salts thereof (e.g., citric acid and sodium citrate). An acid may be selected from citric acid, lactic acid, malic acid, maleic acid, fumaric acid, acetic acid, succinic acid, and tartaric acid. One or more conjugate base salts may be independently selected from sodium and potassium salts, or combinations thereof. Some embodiments optionally comprise citric acid, and the one or more conjugate base salts comprise monosodium citrate (monobasic), disodium citrate (dibasic), tri sodium citrate (tribasic), and combinations thereof. In some embodiments, the optional organic acid buffer system is present in an amount of 0. 1 to 5.0% by weight of the composition, measured as the combined amount of organic acid and any conjugate base salts; for example, from 0.5 to 4.0%, or from 1.0 to 3.0%, or from 1.5 to 3.0%, or from 1.0 to 2.4%, or from 1.0% to 2.0%, or from 1.0% to 1.5%, or about 1.2%, by weight of the composition. In some embodiments, the optional organic acid buffer system consists of an organic acid and a conjugate base salt thereof, for example, in a ratio of from 1:1 to 1:10, e.g., from 1:2 to 1:8, or from 1:3 to 1:6, or from 1:4 to 1:6, or from 1:5 to 1:6, or about 1:5, by weight of the components. In some embodiments, the optional organic acid buffer system comprises citric acid and a sodium citrate salt (e.g., trisodium citrate, disodium citrate, or monosodium citrate), in a ratio of from 1:3 to 1:6, or 1:4 to 1:6, or about 1:5 (e.g., about 1:5.7), by weight.

In some embodiments, the oral care composition further comprises an abrasive, for example, silica abrasives, calcium abrasives, and other abrasives as disclosed herein, and/or one or more humectants and/or one or more surfactants, as described herein and/or an effective amount of one or more alkali phosphate salts for example orthophosphates, pyrophosphates, tripolyphosphates, tetraphosphates or higher polyphosphates. In some embodiments, the alkali phosphate salts comprise tetrasodium pyrophosphate or tetrapotassium pyrophosphate, for example, in an amount of 0.5 to 5% by weight of the composition, e.g., 1-3%, or 1-2% or about 2%> by weight, or about 2-4%, or about 3-4% or about 4% by weight of the composition. In some embodiments, the alkali phosphate salts comprise sodium tripolyphosphate or potassium tripolyphosphate, for example, in an amount of 0.5 to 6% by weight of the composition, e.g., 1-4%, or 2~3%> or about 3%> by weight. Any preceding composition, may further comprise a whitening agent and/or one or more sources of zinc ions in addition to the zinc phosphate, for example a zinc salt selected from zinc citrate, zinc oxide, zinc lactate, zinc pyrophosphate, zinc sulfate, or zinc chloride. In some embodiments, such compositions are dentifrices (e.g., a toothpaste or oral gel), powder (e.g., tooth powder), lozenge, mint, cream, strip or gum (e.g., chewing gum).

In some embodiments, the composition comprises from 0.5 to 3% by weight zinc phosphate; from 0.05 to 11% by weight stannous fluoride; from 1 to 8% by weight alkali phosphate salts selected from sodium phosphate dibasic, potassium phosphate dibasic, dicalcium phosphate dihydrate, tetrasodium pyrophosphate, tetrapotassium pyrophosphate, calcium pyrophosphate, sodium tripolyphosphate, and mixtures of any two or more of these, relative to the weight of the oral care composition, and a silica abrasive. The composition may be essentially free of a halogenated diphenyl ether. The composition may be a single-phase composition or a dual-phase composition. The composition may be free of one or more of zinc oxide, zinc citrate, or zinc lactate. Zinc phosphate may the only zinc ion source. The composition may be essentially free of hexametaphosphate salts (e.g., sodium hexametaphosphate).

Formulations can include stannous levels, provided by stannous fluoride, ranging for example, from 3,000 ppm to 15,000 ppm (mass fraction) stannous ions in the total composition. In embodiments, the soluble stannous content can range from 0.1 wt. % to 0.5 wt. %, or more, such as from 0.15 wt. % to 0.32 wt. %, based on the total weight of the composition.

The compositions may optionally comprise additional ingredients suitable for use in oral care compositions. Examples of such ingredients include active agents, such as a fluoride source and/or a phosphate source in addition to zinc phosphate. The compositions may be formulated in a suitable dentifrice base, e.g., comprising abrasives, e.g., silica abrasives, surfactants, foaming agents, vitamins, polymers, enzymes, humectants, thickeners, additional antimicrobial agents, preservatives, flavorings, colorings, and/or combinations thereof. Examples of suitable dentifrice bases are known in the art. Alternatively, the compositions may be formulated as a gel (e.g., for use in a tray), chewing gum, lozenge or mint. Examples of suitable additional ingredients that can be employed in the compositions of the present disclosure are discussed in more detail below.

Oral care compositions may comprise arginine or a salt thereof. In some embodiments, the arginine is L-arginine or a salt thereof. Suitable salts include salts known in the art to be pharmaceutically acceptable salts are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion. In some embodiments, the arginine in partially or wholly in salt form such as arginine phosphate, arginine hydrochloride or arginine bicarbonate. In some embodiments, the arginine is present in an amount corresponding to 0.1% to 15%, e.g., 0.1 wt. % to 10 wt. %, e.g., 0.1 to 5 wt.%, e.g., 0.5 wt. % to 3 wt. % of the total composition weight, about e.g., 1%, 1.5%, 2%, 3%, 4%, 5%, or 8%, wherein the weight of the arginine is calculated as free form. In some embodiments the arginine is present in an amount corresponding to about 0.5 wt. % to about 20 wt. % of the total composition weight, about 0.5 wt. % to about 10 wt. % of the total composition weight, for example about 1.5 wt. %, about 3.75 wt. %, about 5 wt. %, or about 7.5 wt. % wherein the weight of the arginine is calculated as free form. In some embodiments, the arginine is present in an amount of from 0.5 weight % to 10 weight %, or from 0.5 weight % to 3 weight % or from 1 weight % to 2.85 weight %, or from 1.17 weight % to 2.25 weight %, based or from 1.4 weight % to 1.6 weight %, or from 0.75 weight % to 2.9 weight %, or from 1.3 weight % to 2 weight %, or about 1.5 weight %, based on the total weight of the composition. Typically, the arginine is present in an amount of up to 5% by weight, further optionally from 0.5 to 5% by weight, still further optionally from 2.5 to 4.5% by weight, based on the total weight of the oral care composition. In some embodiments, arginine is present in an amount from 0.1 wt. % - 6.0 wt. %. (e.g., about 1.5 wt. %) or from about 4.5 wt. % - 8.5 wt. % (e.g., 5.0%) or from 3.5 wt. % - 9 wt. % or 8.0 wt. %. In some embodiments, the arginine is present in a dentifrice, at for example about 0.5-2 wt. %, e.g., and about 1% in the case of a mouthwash.

One or more fluoride ion sources are optionally present in an amount providing a clinically efficacious amount of soluble fluoride ion to the oral care composition. A fluoride ion source is useful, for example, as an anti-caries agent. Any orally acceptable particulated fluoride ion source can be used, including stannous fluoride, sodium fluoride, potassium fluoride, potassium monofluorophosphate, sodium monofluorophosphate, ammonium monofluorophosphate, sodium fluorosilicate, ammonium fluorosilicate, indium fluoride, amine fluoride such as olaflur (N'-octadecyltrimethylendiamine-N,N,N'-tris(2-ethanol)-dihydrofluoride), ammonium fluoride, titanium fluoride, hexafluorosulfate, and combinations thereof. Fluoride where present may be present at levels of, e.g., about 25 to about 25,000 ppm, for example about 50 to about 5000 ppm, about 750 to about 2,000 ppm for a consumer toothpaste (e.g., 1000-1500 ppm, e.g., about 1000 ppm, e.g., about 1450ppm)., product. In some embodiments, fluoride is present from about 100 to about 1000, from about 200 to about 500, or about 250 ppm fluoride ion. 500 to 3000 ppm. In some embodiments, the fluoride source provides fluoride ion in an amount of from 50 to 25,000 ppm (e.g., 750 -7000 ppm, e.g., 1000-5500 ppm, e.g., about 500 ppm, 1000 ppm, 1100 ppm, 2800 ppm, 5000 ppm, or 25000 ppm). In some embodiments, the fluoride source is stannous fluoride. In some embodiments, the fluoride source is stannous fluoride which provides fluoride in an amount from 750 - 7000 ppm (e.g., about 1000 ppm, 1100 ppm, 2800 ppm, 5000 ppm). In some embodiments, the fluoride source is stannous fluoride which provides fluoride in an amount of about 5000 ppm. In some embodiments, the fluoride source is sodium fluoride which provides fluoride in an amount from 750 - 2000ppm (e.g., about 1450ppm). In some embodiments, the fluoride source is selected from sodium fluoride and sodium monofluorophosphate and which provides fluoride in an amount from 1000ppm -1500ppm. In some embodiments, the fluoride source is sodium fluoride or sodium monofluorophosphate and which provides fluoride in an amount of about 1450ppm. In some embodiments, stannous fluoride is the only fluoride source. In some embodiments, the fluoride source is stannous fluoride which provides fluoride in an amount from 750 - 7000 ppm (e.g., about 1000 ppm, 1100 ppm, 2800 ppm, 5000 ppm). In some embodiments, the fluoride source is stannous fluoride which provides fluoride in an amount of about 5000 ppm. Fluoride ion sources may be added to the compositions at a level of about 0.001 wt. % to about 10 wt. %, e.g., from about 0.003 wt. % to about 5 wt. %, 0.01 wt. % to about 1 wt., or about 0.05 wt. %. In some embodiment, the stannous fluoride is present in an amount of 0.1 wt. % to 2 wt. % (0.1 wt.% - 0.6 wt. %) of the total composition weight. Fluoride ion sources may be added to the compositions at a level of about 0.001 wt. % to about 10 wt. %, e.g., from about 0.003 wt. % to about 5 wt. %, 0.01 wt. % to about 1 wt., or about 0.05 wt. %. However, it is to be understood that the weights of fluoride salts to provide the appropriate level of fluoride ion will obviously vary based on the weight of the counter ion in the salt, and one of skill in the art may readily determine such amounts. In some embodiment, the fluoride source is a fluoride salt present in an amount of 0.1 wt. % to 2 wt. % (0.1 wt.% - 0.6 wt. %) of the total composition weight (e.g., sodium fluoride (e.g., about 0.32 wt. %) or sodium monofluorophosphate). e.g., 0.3-0.4%, e.g., ca. 0.32% sodium fluoride

The oral care compositions described herein may also comprise one or more further agents such as those typically selected from the group consisting of: abrasives, an anti-plaque agent, a whitening agent, antibacterial agent, cleaning agent, a flavoring agent, a sweetening agent, adhesion agents, surfactants, foam modulators, pH modifying agents, humectants, mouth-feel agents, colorants, tartar control (anti-calculus) agent, polymers, saliva stimulating agent, nutrient, viscosity modifier, anti-sensitivity agent, antioxidant, and combinations thereof.

In some embodiments, the oral care compositions comprise one or more abrasive particulates such as those useful for example as a polishing agent. Any orally acceptable abrasive can be used, but type, fineness, (particle size) and amount of abrasive should be selected so that tooth enamel is not excessively abraded in normal use of the composition. Examples of abrasive particulates may be used include abrasives such sodium bicarbonate, insoluble phosphates (such as orthophosphates, polymetaphosphates and pyrophosphates including dicalcium orthophosphate dihydrate, calcium pyrophosphate, tricalcium phosphate, calcium polymetaphosphate and insoluble sodium polymetaphosphate), calcium phosphate (e.g., dicalcium phosphate dihydrate), calcium sulfate, natural calcium carbonate (CC), precipitated calcium carbonate (PCC), silica (e.g., hydrated silica or silica gels or in the form of precipitated silica or as admixed with alumina), iron oxide, aluminum oxide, aluminum silicate, calcined alumina, bentonite, other siliceous materials, perlite, plastic particles, e.g., polyethylene, and combinations thereof. The natural calcium carbonate abrasive of is typically a finely ground limestone which may optionally be refined or partially refined to remove impurities. The material preferably has an average particle size of less than 10 microns, e.g., 3-7 microns, e.g. about 5.5 microns. For example, a small particle silica may have an average particle size (D50) of 2.5 - 4.5 microns. Because natural calcium carbonate may contain a high proportion of relatively large particles of not carefully controlled, which may unacceptably increase the abrasivity, preferably no more than 0.01%, preferably no more than 0.004%) by weight of particles would not pass through a 325 mesh. The material has strong crystal structure, and is thus much harder and more abrasive than precipitated calcium carbonate. The tap density for the natural calcium carbonate is for example between 1 and 1.5 g/cc, e.g., about 1.2 for example about 1.19 g/cc. There are different polymorphs of natural calcium carbonate, e.g., calcite, aragonite and vaterite, calcite being preferred for purposes of this invention. An example of a commercially available product suitable for use in the present invention includes Vicron ^{®} 25-11 FG from GMZ. Precipitated calcium carbonate has a different crystal structure from natural calcium carbonate. It is generally more friable and more porous, thus having lower abrasivity and higher water absorption. For use in the present invention, the particles are small, e.g., having an average particle size of 1-5 microns, and e.g., no more than 0.1 %, preferably no more than 0.05% by weight of particles which would not pass through a 325 mesh. The particles may for example have a D50 of 3-6 microns, for example 3.8-4.9, e.g., about 4.3; a D50 of 1-4 microns, e.g. 2.2-2.6 microns, e.g., about 2.4 microns, and a D10 of 1-2 microns, e.g., 1.2-1.4, e.g. about 1.3 microns. The particles have relatively high water absorption, e.g., at least 25 g/100 g, e.g. 30-70 g/100 g. Examples of commercially available products suitable for use include, for example, Carbolag^{®} 15 Plus from Lagos Industria Quimica. In some embodiments, additional calcium-containing abrasives, for example calcium phosphate abrasive, e.g., tricalcium phosphate, hydroxyapatite or dicalcium phosphate dihydrate or calcium pyrophosphate, and/or silica abrasives, sodium metaphosphate, potassium metaphosphate, aluminum silicate, calcined alumina, bentonite or other siliceous materials, or combinations thereof are used. Examples of silica abrasives include, but are not limited to, precipitated or hydrated silicas having a mean particle size of up to about 20 microns (such as Zeodent 105 and Zeodent 1 14 marketed by J.M. Huber Chemicals Division, Havre de Grace, Md. 21078); Sylodent 783 (marketed by Davison Chemical Division of W.R. Grace & Company); or Sorbosil AC 43 (from PQ Corporation). In some embodiments, an effective amount of a silica abrasive is about 10-30%, e.g. about 20%. In some embodiments, the acidic silica abrasive Sylodent is included at a concentration of about 2 to about 35% by weight; about 3 to about 20 % by weight, about 3 to about 15% by weight, about 10 to about 15 % by weight. For example, the acidic silica abrasive may be present in an amount selected from 2 wt.%, 3 wt.%, 4 wt.%, 5 wt.%, 6 wt.%, 7 wt.%, 8 wt.%, 9 wt.%, 10 wt.%, 11 wt.%, 12 wt.%, 13 wt.%, 14 wt.%,15 wt.%, 16 wt.%, 17 wt.%, 18 wt.%, 19 wt.%, 20 wt.%. Sylodent 783 has a pH of 3.4-4.2 when measured as a 5% by weight slurry in water and silica material has an average particle size of less than 10 microns, e.g., 3-7 microns, e.g. about 5.5 microns. In some embodiments, the silica is synthetic amorphous silica, (e.g., 1% - 28% by wt.) (e.g., 8% - 25% by wt.). In some embodiments, the silica abrasives are silica gels or precipitated amorphous silicas, e.g. silicas having an average particle size ranging from 2.5 microns to 12 microns. Some embodiments further comprise a small particle silica having a median particle size (d50) of 1- 5 microns (e.g., 3 - 4 microns) (e.g., about 5 wt. % Sorbosil AC43 from PQ Corporation Warrington, United Kingdom). The composition may contain from 5 to 20 wt. % small particle silica, or for example 10 - 15 wt. %, or for example 5 wt. %, 10 wt.%, 15 wt. % or 20 wt. % small particle silica. In some embodiments, 20-30 wt.% of the total silica in the composition is small particle silica (e.g., having a median particle size (d50) of 3-4 microns and wherein the small particle silica is about 5 wt. % of the oral care composition. In some embodiments, silica is used as a thickening agent, e.g., particle silica. In some embodiments, the composition comprises calcium carbonate, such as precipitated calcium carbonate high absorption (e.g., 20% to 30% by weight of the composition or, 25% precipitated calcium carbonate high absorption), or precipitated calcium carbonate - light (e.g., about 10% precipitated calcium carbonate - light) or about 10% natural calcium carbonate.

In some embodiments, the oral care compositions comprise a whitening agent, e.g., a selected from the group consisting of peroxides, metal chlorites, perborates, percarbonates, peroxyacids, hypochlorites, hydroxyapatite, and combinations thereof. Oral care compositions may comprise hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example, calcium peroxyphosphate, sodium perborate, sodium carbonate peroxide, sodium peroxyphosphate, and potassium persulfate or hydrogen peroxide polymer complexes such as hydrogen peroxide-polyvinyl pyrrolidone polymer complexes.

In some embodiments, the oral care compositions comprise an effective amount of one or more antibacterial agents, for example comprising an antibacterial agent selected from halogenated diphenyl ether (e.g. triclosan), triclosan monophosphate, herbal extracts and essential oils (e.g., rosemary extract, tea extract, magnolia extract, thymol, menthol, eucalyptol, geraniol, carvacrol, citral, hinokitol, magonol, ursolic acid, ursic acid, morin, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine furanones, bacteriocins, ethyllauroyl arginate, arginine bicarbonate, a Camellia extract, a flavonoid, a flavan, halogenated diphenyl ether, creatine, sanguinarine, povidone iodine, delmopinol, salifluor, metal ions (e.g., zinc salts, stannous salts, copper salts, iron salts), propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nisin preparations, chlorite salts; parabens such as methylparaben or propylparaben and mixtures of any of the foregoing. One or more additional antibacterial or preservative agents may optionally be present in the composition in a total amount of from about 0.01 wt. % to about 0.5 wt. %, optionally about 0.05 wt. % to about 0.1 wt. % or about 0.3%.by total weight of the composition.

In some embodiments, the oral care compositions may comprise at least one bicarbonate salt useful for example to impart a "clean feel" to teeth and gums due to effervescence and release of carbon dioxide. Any orally acceptable bicarbonate can be used, including without limitation, alkali metal bicarbonates such as sodium and potassium bicarbonates, ammonium bicarbonate and the like. The one or more additional bicarbonate salts are optionally present in a total amount of about 0.1 wt. % to about 50 wt. %, for example about 1 wt. % to 20 wt. %, by total weight of the composition.

In some embodiments, the oral care compositions also comprise at least one flavorant, useful for example to enhance taste of the composition. Any orally acceptable natural or synthetic flavorant can be used, including without limitation essential oils and various flavoring aldehydes, esters, alcohols, and similar materials, tea flavors, vanillin, sage, marjoram, parsley oil, spearmint oil, cinnamon oil, oil of wintergreen, peppermint oil, clove oil, bay oil, anise oil, eucalyptus oil, citrus oils, fruit oils, sassafras and essences including those derived from lemon, orange, lime, grapefruit, apricot, banana, grape, apple, strawberry, cherry, pineapple, etc., bean- and nut- derived flavors such as coffee, cocoa, cola, peanut, almond, etc., adsorbed and encapsulated flavorants and the like. Also encompassed within flavorants herein are ingredients that provide fragrance and/or other sensory effect in the mouth, including cooling or wanning effects. Such ingredients illustratively include menthol, carvone, menthyl acetate, menthyl lactate, camphor, eucalyptus oil, eucalyptol, anethole, eugenol, cassia, oxanone, a-irisone, propenyl guaiethoi, thymol, linalool, benzaldehyde, cinnamaldehyde, N-ethyl-p-menthan-3-carboxamine, N,2,3-trimethyl-2- isopropylbutanamide, 3-(1-menthoxy)-propane-1,2-diol, cinnamaldehyde glycerol acetal (CGA), menthone glycerol acetal (MGA) and the like. One or more flavorants are optionally present in a total amount of from about 0.01 wt. % to about 5 wt. %, for example, from about 0.03 wt. % to about 2.5 wt.%, optionally about 0.05 wt.% to about 1.5 wt.%, further optionally about 0.1 wt.% to about 0.3 wt.% and in some embodiments in various embodiments from about 0.01 wt. % to about 1 wt. %, from about 0.05 to about 2%, from about 0.1% to about 2.5%, and from about 0.1 to about 0.5% by total weight of the composition.

In some embodiments, the oral care compositions comprise at least one sweetener, useful for example to enhance taste of the composition. Sweetening agents among those useful herein include dextrose, polydextrose, sucrose, maltose, dextrin, dried invert sugar, mannose, xylose, ribose, fructose, levulose, galactose, corn syrup, partially hydrolyzed starch, hydrogenated starch hydrolysate, ethanol, sorbitol, mannitol, xylitol, maltitol, isomalt, aspartame, neotame, saccharin and salts thereof (e.g. sodium saccharin), sucralose, dipeptide-based intense sweeteners, cyclamates, dihydrochalcones, glycerine, propylene glycol, polyethylene glycols, Poloxomer polymers such as POLOXOMER 407, PLURONIC F108, (both available from BASF Corporation), alkyl polyglycoside (APG), polysorbate, PEG40, castor oil, menthol, and mixtures thereof. One or more sweeteners are optionally present in a total amount depending strongly on the particular sweetener(s) selected, but typically 0.005 wt.% to 5 wt.%, by total weight of the composition, optionally 0.005 wt.% to 0.2 wt.%, further optionally 0.05 wt.% to 0.1 wt.% by total weight of the composition.

In some embodiments, the oral care compositions further comprise an agent that interferes with or prevents bacterial attachment, e.g., ethyl lauroyl arginate (ELA), solbrol or chitosan, as well as plaque dispersing agents such as enzymes (papain, glucoamylase, etc.).

In some embodiments, the oral care compositions also comprise at least one surfactant. Any orally acceptable surfactant, most of which are anionic, cationic, zwitterionic, nonionic or amphoteric, and mixtures thereof, can be used. Examples of suitable surfactants include water-soluble salts of higher fatty acid monoglyceride monosulfates, such as the sodium salt of monosulfated monoglyceride of hydrogenated coconut oil fatty acids; higher alkyl sulfates such as sodium lauryl sulfate, sodium coconut monoglyceride sulfonate, sodium lauryl sarcosinate, sodium lauryl isoethionate, sodium laureth carboxylate and sodium dodecyl benzenesulfonate; alkyl aryl sulfonates such as sodium dodecyl benzene sulfonate; higher alkyl sulfoacetates, such as sodium lauryl sulfoacetate; higher fatty acid esters of 1,2-dihydroxypropane sulfonate; and the substantially saturated higher aliphatic acyl amides of lower aliphatic amino carboxylic compounds, such as those having 12-16 carbons in the fatty acid, alkyl or acyl radicals; and the like. Examples of amides include N-lauryl sarcosine, and the sodium, potassium and ethanolamine salts of N-lauryl, N-myristoyl, or N-palmitoyl sarcosine. Examples of cationic surfactants include derivatives of aliphatic quaternary ammonium compounds having one long alkyl chain containing 8 to 18 carbon atoms such as lauryl trimethylammonium chloride, cetyl pyridinium chloride, cetyl trimethyl ammonium bromide, di-isobutylphenoxyethyldimethylbenzylammonium chloride, coconut alkyltrimethylammonium nitrite, cetyl pyridinium fluoride, and mixtures thereof. Suitable nonionic surfactants include without limitation, poloxamers, polyoxyethylene sorbitan esters, fatty alcohol ethoxylates, alkylphenol ethoxylates, tertiary amine oxides, tertiary phosphine oxides, di alkyl sulfoxides and the like. Others include, for example, non-anionic polyoxyethylene surfactants, such as Polyoxamer 407, Steareth 30, Polysorbate 20, and castor oil; and amphoteric surfactants such as derivatives of aliphatic secondary and tertiary amines having an anionic group such as carboxylate, sulfate, sulfonate, phosphate or phosphonate such as cocamidopropyl betaine (tegobaine), and cocamidopropyl betaine lauryl glucoside; condensation products of ethylene oxide with various hydrogen containing compounds that are reactive therewith and have long hydrocarbon chains (e.g., aliphatic chains of from 12 to 20 carbon atoms), which condensation products (ethoxamers) contain hydrophilic polyoxyethylene moieties, such as condensation products of poly (ethylene oxide) with fatty acids, fatty, alcohols, fatty amides and other fatty moieties, and with propylene oxide and polypropylene oxides. In some embodiments, the oral composition includes a surfactant system that is sodium laurel sulfate (SLS) and cocamidopropyl betaine. One or more surfactants are optionally present in a total amount of about 0.01 wt.% to about 10 wt. %, for example, from about 0.05 wt. % to about 5 wt. %, or from about 0.1 wt. % to about 2 wt. %, e.g. 1.5% wt. by total weight of the composition. In some embodiments, the oral composition include an anionic surfactant, e.g., a surfactant selected from sodium lauryl sulfate, sodium ether lauryl sulfate, and mixtures thereof, e.g. in an amount of from about 0.3% to about 4.5% by weight, e.g. 1-2% sodium lauryl sulfate (SLS); and/or a zwitterionic surfactant, for example a betaine surfactant, for example cocamidopropylbetaine, e.g. in an amount of from about 0.1% to about 4.5% by weight, e.g. 0.5-2% cocamidopropylbetaine. Some embodiments comprise a nonionic surfactant in an amount of from 0.5 -5%, e.g, 1-2%, selected from poloxamers (e.g., poloxamer 407), polysorbates (e.g., polysorbate 20), polyoxyl hydrogenated castor oil (e.g., polyoxyl 40 hydrogenated castor oil), and mixtures thereof. In some embodiments, the poloxamer nonionic surfactant has a polyoxypropylene molecular mass of from 3000 to 5000 g/mol and a polyoxyethylene content of from 60 to 80 mol%, e.g., the poloxamer nonionic surfactant comprises poloxamer 407. Any of the preceding compositions may further comprise sorbitol, wherein the sorbitol is in a total amount of 10- 40% (e.g., about 23%).

In some embodiments, the oral care compositions comprise at least, one foam modulator, useful for example to increase amount, thickness or stability of foam generated by the composition upon agitation. Any orally acceptable foam modulator can be used, including without limitation, polyethylene glycols (PEGs), also known as polyoxyethylenes. High molecular weight PEGs are suitable, including those having an average molecular weight of 200,000 to 7,000,000, for example 500,000 to 5,000,000, or 1,000,000 to 2,500,000, One or more PEGs are optionally present in a total amount of about 0.1 wt. % to about 10 wt. %, for example from about 0.2 wt. % to about 5 wt. %, or from about 0.25 wt. % to about 2 wt.%, by total weight of the composition

In some embodiments, the oral care compositions comprise at least one pH modifying agent. Such agents include acidifying agents to lower pH, basifying agents to raise pH, and buffering agents to control pH within a desired range. For example, one or more compounds selected from acidifying, basifying and buffering agents can be included to provide a pH of 2 to 10, or in various illustrative embodiments, 2 to 8, 3 to 9, 4 to 8, 5 to 7, 6 to 10, 7 to 9, etc. Any orally acceptable pH modifying agent can be used, including without limitation, carboxylic, phosphoric and sulfonic acids, acid salts (e.g., monosodium citrate, disodium citrate, monosodium malate, etc.), alkali metal hydroxides such as sodium hydroxide, carbonates such as sodium carbonate, bicarbonates such as sodium bicarbonate, sesquicarbonates, borates, silicates, bisulfates, phosphates (e.g., monosodium phosphate, trisodium phosphate, monopotassium phosphate, dipotassium phosphate, tribasic sodium phosphate, sodium tripolyphosphate, phosphoric acid), imidazole, sodium phosphate buffer (e.g., sodium phosphate monobasic and disodium phosphate) citrates (e.g. citric acid, trisodium citrate dehydrate), pyrophosphates (sodium and potassium salts) and the like and combinations thereof. One or more pH modifying agents are optionally present in a total amount effective to maintain the composition in an orally acceptable pH range. Compositions may have a pH that is either acidic or basic, e.g., from pH 4 to pH 5.5 or from pH 8 to pH 10. In some embodiments, the amount of buffering agent is sufficient to provide a pH of about 5 to about 9, preferable about 6 to about 8, and more preferable about 7, when the composition is dissolved in water, a mouth rinse base, or a toothpaste base. Typical amounts of buffering agent are about 5% to about 35%, in one embodiment about 10% to about 30%), in another embodiment about 15% to about 25%, by weight of the total composition.

In some embodiments, the oral care compositions also comprise at least one humectant. Any orally acceptable humectant can be used, including without limitation, polyhydric alcohols such as glycerin, sorbitol (optionally as a 70 wt. % solution in water), propylene glycol, xylitol or low molecular weight polyethylene glycols (PEGs) and mixtures thereof. Most humectants also function as sweeteners. In some embodiments, compositions comprise 15% to 70% or 30% to 65% by weight humectant. Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the compositions herein. One or more humectants are optionally present in a total amount of from about 1 wt.% to about 70 wt.%, for example, from about 1 wt.% to about 50 wt.%, from about 2 wt.% to about 25 wt.%, or from about 5 wt.% to about 15 wt.%, by total weight of the composition. In some embodiments, humectants, such as glycerin are present in an amount that is at least 20%>, e.g., 20-40%, e.g., 25-35%.

Mouth-feel agents include materials imparting a desirable texture or other feeling during use of the composition. In some embodiments, the oral care compositions comprise at least one thickening agent, useful for example to impart a desired consistency and/or mouth feel to the composition. Any orally acceptable thickening agent can be used, including without limitation, carbomers, also known as carboxyvinyl polymers, carrageenans, also known as Irish moss and more particularly i-carrageenan (iota-carrageenan), cellulosic polymers such as hydroxyethyl cellulose, and water-soluble salts of cellulose ethers (e.g., sodium carboxymethyl cellulose and sodium carboxymethyl hydroxyethyl cellulose), carboxymethylcellulose (CMC) and salts thereof, e.g., CMC sodium, natural gums such as karaya, xanthan, gum arabic and tragacanthin, colloidal magnesium aluminum silicate, colloidal silica, starch, polyvinyl pyrrolidone, hydroxyethyl propyl cellulose, hydroxybutyl methyl cellulose, hydroxypropyl methyl cellulose, and hydroxyethyl cellulose and amorphous silicas, and the like. A preferred class of thickening or gelling agents includes a class of homopolymers of acrylic acid crosslinked with an alkyl ether of pentaerythritol or an alkyl ether of sucrose, or carbomers. Carbomers are commercially available from B. F. Goodrich as the Carbopol^{©} series. Particularly preferred Carbopols include Carbopol 934, 940, 941, 956, 974P, and mixtures thereof. Silica thickeners such as DT 267 (from PPG Industries) may also be used. One or more thickening agents are optionally present in a total amount of from about 0.01 wt. % to 15 wt.%, for example from about 0.1 wt.% to about 10 wt.%, or from about 0.2 wt. % to about 5 wt.%, by total weight of the composition. Some embodiments comprise sodium carboxymethyl cellulose (e.g., from 0.5 wt. % - 1.5 wt. %). In certain embodiments, thickening agents in an amount of about 0.5% to about 5.0% by weight of the total composition are used. Thickeners may be present in an amount of from 1 wt. % to 15 wt. %, from 3 wt. % to 10 wt. %, 4 wt. % to 9 wt. %, from 5 wt. % to 8 wt. %, for example 5 wt. %, 6 wt. %, 7 wt. %, or 8 wt. %.

In some embodiments, the oral care compositions comprise at least one colorant. Colorants herein include pigments, dyes, lakes and agents imparting a particular luster or reflectivity such as pearling agents. In various embodiments, colorants are operable to provide a white or light-colored coating on a dental surface, to act as an indicator of locations on a dental surface that have been effectively contacted by the composition, and/ or to modify appearance, in particular color and/ or opacity, of the composition to enhance attractiveness to the consumer. Any orally acceptable colorant can be used, including FD&C dyes and pigments, talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride, and mixtures thereof. One or more colorants are optionally present in a total amount of about 0.001% to about 20%, for example about 0.01% to about 10% or about 0.1% to about 5% by total weight of the composition.

In some embodiments, the oral care composition further comprises an anti-calculus (tartar control) agent. Suitable anti-calculus agents include, but are not limited to: phosphates and polyphosphates, polyaminopropane sulfonic acid (AM PS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates. Soluble pyrophosphates are useful anticalculus agents. The pyrophosphate salts can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The pyrophosphates also contribute to preservation of the compositions by lowering water activity, tetrasodium pyrophosphate (TSPP), tetrapotassium pyrophosphate, sodium tripolyphosphate, tetrapolyphosphate, sodium trimetaphosphate, sodium hexametaphosphate and mixtures thereof. The salts are useful in both their hydrated and unhydrated forms. An effective amount of pyrophosphate salt useful in the present composition is generally enough to provide least 0.1 wt. % pyrophosphate ions, e.g., 0.1 to 3 wt. %, e.g., 0.1 to 2 wt. %, e.g., 0.1 to 1 wt. %, e.g., 0.2 to 0.5 wt. %.

Other useful tartar control agents include polymers and co-polymers. In some embodiments, the oral care compositions include one or more polymers, such as polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water-soluble alkali metal (e.g., potassium and sodium) or ammonium salts. Certain embodiments include 1:4 to 4:1 copolymers of maleic anhydride or acid with another polymerizable ethylenically unsaturated monomer, for example, methyl vinyl ether (methoxyethylene), having a molecular weight (M.W.) of about 30,000 to about 1,000,000, polyvinyl methyl ether/maleic anhydride (PVM/MA) copolymers such as GANTREZ^{®} (e.g., GANTREZ^{®} S-97 polymer). In some embodiments, the PVM/MA copolymer comprises a copolymer of methyl vinyl ether/maleic anhydride, wherein the anhydride is hydrolyzed following copolymerization to provide the corresponding acid. In some embodiments, PVM/MA copolymer has an average molecular weight (M.W.) of about 30,000 to about 1,000,000, e.g. about 300,000 to about 800,000, e.g., wherein the anionic polymer is about 1-5%, e.g., about 2%, of the weight of the composition. In some embodiments, the anti-calculus agent is present in the composition in an amount of from 0.2 weight % to 0.8 weight %; 0.3 weight % to 0.7 weight %; 0.4 weight % to 0.6 weight %; or about 0.5 weight %, based on the total weight of the composition. Copolymers are available for example as Gantrez AN 139(M.W. 500,000), AN 1 19 (M.W. 250,000) and S-97 Pharmaceutical Grade (M.W. 70,000), of GAF Chemicals Corporation. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethyl enically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha- chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000. Another useful class of polymeric agents includes polyamino acids, particularly those containing proportions of anionic surface-active amino acids such as aspartic acid, glutamic acid and phosphoserine.

In some embodiments, the oral care compositions comprise a saliva stimulating agent useful, for example, in amelioration of dry mouth. Any orally acceptable saliva stimulating agent can be used, including without limitation food acids such as citric, lactic, malic, succinic, ascorbic, adipic, fumaric and tartaric acids, and mixtures thereof. One or more saliva stimulating agents are optionally present in saliva stimulating effective total amount.

In some embodiments, the oral care compositions comprise a nutrient. Suitable nutrients include vitamins, minerals, amino acids, and mixtures thereof. Vitamins include Vitamins C and D, miamine, riboflavin, calcium pantothenate, niacin, folic acid, nicotinamide, pyridoxine, cyanocobalamin, para-aminobenzoic acid, bioflavonoids, and mixtures thereof. Nutritional supplements include amino acids (such as L-tryptophan, L-lysine, methionine, threonine, levocarnitine and L-carnitine), lipotropics (such as choline, inositol, betaine, and linoleic acid), and mixtures thereof.

In some embodiments, the oral care compositions comprise at least one viscosity modifier, useful for example to help inhibit settling or separation of ingredients or to promote redispersibility upon agitation of a liquid composition. Any orally acceptable viscosity modifier can be used, including without limitation, mineral oil, petrolatum, clays and organo-modified clays, silicas and the like. One or more viscosity modifiers are optionally present in a total amount of from about 0.01 wt. % to about 10 wt. %, for example, from about 0.1 wt.% to about 5 wt.%, by total weight of the composition.

In some embodiments, the oral care compositions comprise antisensitivity agents, e.g., potassium salts such as potassium nitrate, potassium bicarbonate, potassium chloride, potassium citrate, and potassium oxalate; capsaicin; eugenol; strontium salts; chloride salts and combinations thereof. Such agents may be added in effective amounts, e.g., from about 1 wt. % to about 20 wt. % by weight based on the total weight of the composition, depending on the agent chosen.

In some embodiments, the oral care compositions comprise an antioxidant. Any orally acceptable antioxidant can be used, including butylated hydroxy anisole (BHA), butylated hydroxytoluene (BHT), vitamin A, carotenoids, co-enzyme Q10, PQQ, Vitamin A, Vitamin C, vitamin E, anethole-dithiothione, flavonoids, polyphenols, ascorbic acid, herbal antioxidants, chlorophyll, melatonin, and mixtures thereof.

In some embodiments, the oral care compositions comprise a source of calcium and phosphate selected from (i) calcium-glass complexes, e.g., calcium sodium phosphosilicates, and (ii) calcium-protein complexes, e.g., casein phosphopeptide-amorphous calcium phosphate. Any of the preceding compositions further comprising a soluble calcium salt, e.g., selected from calcium sulfate, calcium chloride, calcium nitrate, calcium acetate, calcium lactate, and combinations thereof.

In some embodiments, the oral care compositions comprise an additional ingredient selected from: benzyl alcohol, Methylisothizolinone ("MIT"), Sodium bicarbonate, sodium methyl cocoyl taurate (tauranol), lauryl alcohol, and polyphosphate. Some embodiments comprise benzyl alcohol that is present from 0.1 - 0.8 wt. %., or 0.2 to 0.7 wt. %, or from 0.3 to 0.6 wt. %, or from 0.4 to 0.5 wt. %, e.g. about 0.1 wt. %, about 0.2 wt. %, about 0.3 wt. %, about 0.4 wt. %, about 0.5 wt. %, about 0.6 wt.%, about 0.7 wt. % or about 0.8 wt. %.

### EXAMPLES

### Example 1

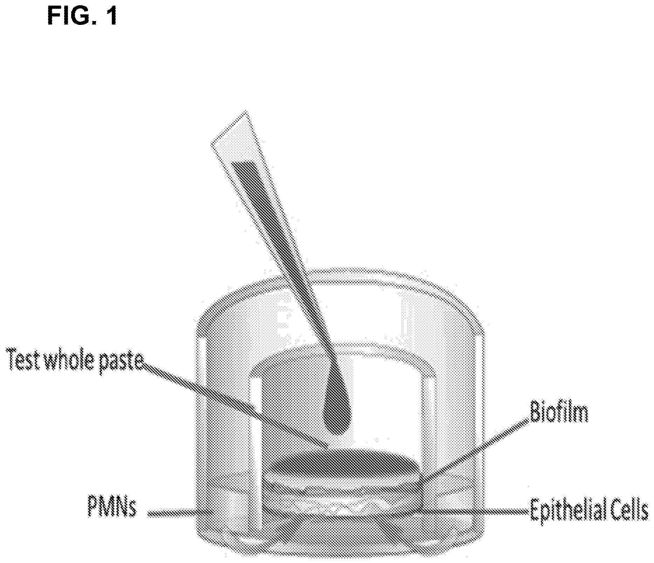

Figure 1 contains an illustration of the Gingival Crevice Model (GCM). The GCM is useful to assess product health benefits in a cell culture model that closely mimics a gingival crevice. The gingival crevice is home to hundreds of bacterial species along with gingival epithelial cells and neutrophils. Proteomics of secreted or expressed proteins, bacterial impact and odor can be evaluated and used to compare the impact of various compounds and compositions. The GCM combines three components, neutrophil-like cells, biofilm that includes oral bacteria, and oral epithelial tissue.

Neutrophil-like cells: HL60 cells (ATCC #CCLO-240) can be induced to differentiate into a neutrophil-like cell types by contacting the HL60 cells with retinoic acid. HL60 cells are maintained at a cell density of 1×10⁵ cells/mL (Media for HL60 IMEM ATCC #30-2005). Retinoic acid for differentiation of HL60s into neutrophil-like is prepared by dissolving retinoic acid into ETOH to produce a 1 mM solution of retinoic acid in ethanol. When the HL60 cells are to be induced to differentiate into a neutrophil-like cell types by retinoic acid at a concentration of 1 µM (1:1000 dilution of the 1mM retinoic acid solution), the HL60 cells are brought up to a cell density of 2×10⁵ cells/mL. Differentiation takes 6 days. Differentiated cells, which make up about 60-80% of cells and are referred to in Figure 1 as PMNs.

Biofilm: Biofilms are created using saliva cultivated on substrates such as HAP discs, poly-D-lysine, or collagen-coated substrates, or *in vivo* using enamel in an individually made retainer, collagen matrices, and polydimethylsiloxane (PDMS), agarose, agar, poly(ethylene glycol) dimethacrylate (PEGDMA) and 2-methacryloyloxyethyl phosphorylcholine polymer (PMPC) hydrogels. The cultivation of biofilm typically takes 2 days. McBain media supplemented with 5 µg/ml hemin (final concentration) and 1 µg/ml (final concentration) is inoculated with ~2 mL of human saliva. Salivary biofilms are cultured for ~16 hours on substrates, for example HAP disks, under suitable growing conditions such as 37°C under 5% CO².

Oral Epithelial Tissue: There are two types of oral tissue available from MatTek: EpiGingival^{™} gingival epithelium and EpiOral^{™} oral (buccal) epithelium. MatTek's EpiOral and EpiGingival tissues consist of normal, human-derived oral epithelial cells. The cells have been cultured to form multilayered, highly differentiated models of the human buccal (EpiOral) and gingival (EpiGingival) phenotypes. The tissues are cultured on specially prepared cell culture inserts using serum free medium. The EpiOral and EpiGingival tissue models exhibit *in* vivo-like morphological and growth characteristics which are uniform and highly reproducible. For traditional GCM, the gingival epithelium is preferred. If a cheek model is the goal, the Oral epithelium is used.

Prior to assembly in the GCM, the HL60 cells must be induced with the retinoic acid to differentiate into the neutrophil-like phenotype (PMNs) and the biofilms must be prepared. The preparation of PMNs and biofilms are coordinated so that the PMNs and biofilms are ready following receipt from the supplier and overnight incubation of the MatTek tissue. Upon delivery of the MatTek tissue (epithelial cells) is placed in fresh media in 6 well plates and left to recover overnight in incubator.

On testing day, the preparation of each of the components of the GCM is coordinated so the each of the components of the GCM is ready for testing at the same time.

When testing toothpaste (TP), the product is prepared as a slurry. The TP product is diluted with ultrapure H₂O immediately prior to testing at 1:2 dilution. Mouthwash can be used at full strength.

MatTek media and (FBS) serum are warmed. Tissue and biofilm are treated separately and the GCM is assembled.

Biofilms are treated once with the 1:2 (TP:water) toothpaste slurry for 2 minutes at room temperature while shaking at ~100 rpm. Following treatment, the biofilms are washed twice in sterile deionized water at 5 minute intervals and then transferred into fresh sterile water to allow the bacteria to recover at 37°C for ~3 hours prior to assembly of the GCM and co-incubation with treated cultured epithelial cells.

To treat the MatTek epithelial tissue, the MatTek tissue is removed from the incubator, and each tissue is taken out for treatment with the 1:2 (TP:water) toothpaste slurry in a 24 well plate. Prior to treatment the media is removed for use a baseline control. Each tissue sample is treated with toothpaste dilution for 2 minutes.

Differentiated HL60s (2.5 × 10⁵cells/mL) are prepared for the GCM by centrifuging 300 RPM for 5 minutes in fresh tubes and re-suspending in MatTek media to model a non-inflammatory condition or MatTek + 5% FBS to model an inflammatory condition.

Biofilm and epithelial tissue, which have each been treated with the same type of tooth paste dilution, and PMNs are assembles as shown in Figure 1 and placed in a bacteria-friendly incubator overnight.

After 24 hours, media from experiment is harvested and HL60s/PMNs are spun out (300RPM, 5min) and frozen/store at -20°C. Cytokine/chemokines are detected and quantified using Milliplex MagPix kits. Bacterial analysis can be performed on biofilms on HAP discs or other substrates. Alternatively, the biofilms can be stored in -80°C for later analysis.

PMNs can be recovered for analysis. After removing supernatant from cells, the cells are washed two times in cold PBS (300RPM, 5min). The PMNs are brought up in 200uL of fixation buffer (room temp for 10min or overnight at 4°C) and stained with desired antibody staining procedure.

MatTek tissue can be evaluated after treatment. MTT assay should be done if there is question about cellular toxicity. The tissue is fixed for histological analysis if the location of protein expression is to be assessed. Tissue may be sonicated and analyzed for cytokine analysis if the protein of interest is not secreted.

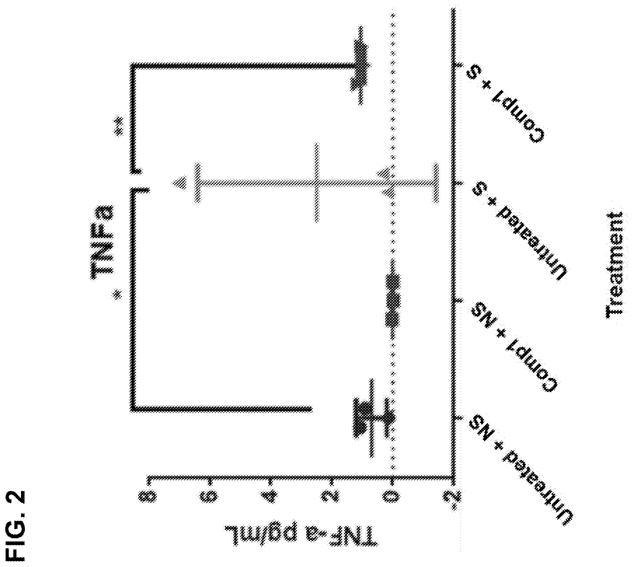

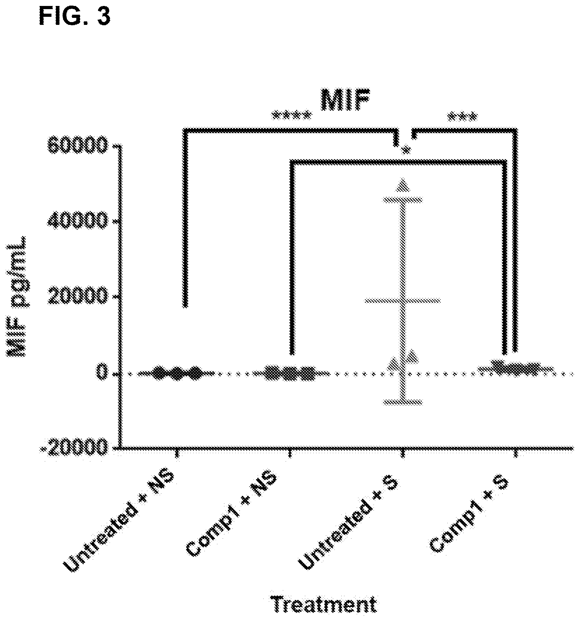

The GCM was used to evaluate toothpaste composition, Composition 1 (Comp1) that comprises stannous fluoride (0.454% SnF₂) and zinc phosphate (1.0% Zn₃(PO₄)₂). Data from the GCM experiment is shown in Figures 2 and 3. TNFa (Figure 2) and MIF (Figure 3) levels in response to the treatment with the test toothpaste were measured and compared to results from untreated controls.

### Example 2

Oral compositions that comprise arginine are disclosed in WO 2017/223292. In some embodiments, the oral care composition comprises an orally acceptable carrier, zinc phosphate; and stannous fluoride. In some embodiments, the zinc phosphate is a preformed salt of zinc phosphate. In some embodiments, the zinc phosphate is present in an amount sufficient so that the stannous fluoride dissociates to provide a therapeutically effective amount of stannous ions in aqueous solution. In some embodiments, the amount of zinc phosphate is from 0.05 to 5% by weight, relative to the weight of the oral care composition. In some embodiments, the amount of the stannous fluoride is from 0.05% to 5% by weight relative to the weight of the oral care composition. In some embodiments, the amount of the water is about 12% by weight or more, relative to the weight of the oral care composition. In some embodiments, the oral care composition further comprises an abrasive and/or one or more humectants and/or one or more surfactants. In some embodiments, the oral care composition further comprises an effective amount of one or more alkali phosphate salts and/or a whitening agent. In some embodiments, the oral care composition further comprising one or more sources of zinc ions in addition to the zinc phosphate. In some embodiments, the oral care composition is a dentifrice, powder, cream, strip, gum or gel. In some embodiments, the oral care composition comprises: from 0.5 to 3% by weight zinc phosphate; from 0.05 to 1 1% by weight stannous fluoride; from 1 to 8% by weight alkali phosphate salts selected from sodium phosphate dibasic, potassium phosphate dibasic, dicalcium phosphate dihydrate, tetrasodium pyrophosphate, tetrapotassium pyrophosphate, calcium pyrophosphate, sodium tripolyphosphate, and mixtures of any two or more of these, relative to the weight of the oral care composition; and a silica abrasive. In some embodiments, the oral care composition has a pH that is less than 7.

### Example 3

Oral compositions that comprise arginine are disclosed in WO 2017/223311. The oral care composition is an oral care composition set out in Example 2 that further comprises an organic acid buffer system. In some embodiments, the oral care composition comprises an amount of the water that is 10% by weight or more, relative to the weight of the oral care composition. In some embodiments, the organic buffer system comprises a carboxylic acid and one or more conjugate base salts thereof, for example, alkali metal salts thereof. In some embodiments, the acid is selected from citric acid, lactic acid, malic acid, maleic acid, fumaric acid, acetic acid, succinic acid, and tartaric acid. In some embodiments, the one or more conjugate base salts are independently selected from sodium and potassium salts, or combinations thereof. In some embodiments, the acid is citric acid, and the one or more conjugate base salts comprise monosodium citrate (monobasic), di sodium citrate (dibasic), trisodium citrate (tribasic), and combinations thereof. In some embodiments, the oral care compositions comprise the organic acid buffer system in an amount of 0. 1 to 5.0% by weight of the composition, measured as the combined amount of organic acid and any conjugate base salt. In some embodiments, the buffer system comprises citric acid and a sodium citrate salt, in a ratio of from 1:3 to 1:6.

### Example 4

Test dentifrices comprising zinc phosphate and stannous fluoride were prepared as shown in Formulation Tables A-D

**Formulation Table A**

| Ingredient | |
|---|---|
| Water | QS (e.g. 15-40) |
| Thickener | 0.5-5 (e.g. 3.6) |
| Humectants | *15-55* (e.g. 48) |
| Tarter control agents | 0.5-5 (e.g. 2) |
| Abrasives | 10-30 (e.g. 20) |
| Stannous Fluoride | 0.5-11 (e.g. 0.454) |
| Minors (flavor, color) | 0.5-5 (e.g. 2.25) |
| Surfactants | 0.1-15 (e.g. 2.75) |
| Zinc phosphate | 0.5-5 (e.g. 1 or 2) |

**Formulation Table B**

| Ingredient | |
|---|---|
| Water and Minors (flavor, color) | 11.74 |
| Stannous Fluoride | 0.454 |
| Zinc phosphate | 1.15 |
| Thickener | 2.9 |
| Glycerin | 40.79 |
| Abrasive Silica | 24.00 |
| Propylene glycol | 4.00 |
| Trisodium citrate trihydrate | 3.00 |
| Sodium tripolyphosphate | 3.00 |
| Polyethylene glycol 600 | 3.00 |
| Tetrasodium pyrophosphate | 2.00 |
| Anionic Surfactant | 1.75 |
| Zwitterionic Surfactant | 1.0 |
| Anionic polymer | 0.61 |
| Citric acid | 0.60 |

**Formulation Table C**

| Ingredient | |
|---|---|
| Zinc phosphate | 0.5-2.5 (e.g. about 1) |
| Stannous Fluoride | 0.3-1 (e.g. about 0.45) |
| Alkali metal pyrophosphate (Tetrapotassium pyrophosphate, Tetrasodium pyrophosphate) | 1-5 (e.g. about 2 or 4) |
| Sodium citrate (Trisodium citrate dihydrate) | 0.8-2.5 (e.g. about 1) |
| Citric Acid | 0.15-0.5 (e.g. about 0.2) |
| Anionic Surfactant (sodium lauryl sulfate) | 1-3 (e.g. about 1.5) |
| Zwitterionic Surfactant (CAPB) | 1-3 (e.g. about 1.25) |
| Sorbitol (e.g. 70% sorbitol) | 20-50 (e.g. about 40) |
| Glycerin | 1-8 (e.g. about 4) |
| Gum polymer (xanthan gum) | 0.5-2 (e.g. about 0.3) |
| Polyethylene glycol (PEG 600) | 1-5 (e.g. about 2) |
| Carboxymethyl cellulose (NaCMC) | 0.5-3 (e.g. about 2) |
| Water (added water) | 10-30, 15-20 (e.g. about 20), 20-50 (e.g. about 30) |

**Formulation Table D**

| Ingredient | | |
|---|---|---|
| Water | QS (e.g. 15-40) | QS (e.g. 15-25) |
| Humectants | *15-55* (e.g. 40) | 40 |
| Abrasives | 10-30 (e.g. 20) | 20 |
| Thickeners | 0.5-5 (e.g. 3.6) | 3.6 |
| Organic acid buffer salt (Trisodium citrate) | 0.0-0.6 | 0.0-0.6 |
| Zinc phosphate | 0.5-5 (e.g. 2.3) | 2.3 |
| Flavors, sweeteners, colors | 0.5-5 (e.g. 0.65) | 0.65 |
| Alkali phosphate salts | 0.5-5 (e.g. 2) | 2 |
| Anionic Surfactant | 0.01-10 (e.g. 1.5) | 1.5 |
| Zwitterionic Surfactant | 0.01-4.5 (e.g. 1.25) | 1.25 |
| Organic acid buffer acid | 0.0-0.3 | 0.0-0.3 |
| Stannous Fluoride | 0.5-11 (e.g. 0.454) | 0.454 |

## Claims

1. An oral care composition comprising: zinc phosphate, stannous fluoride and optionally, an organic acid buffer system for use in a method of increasing proteins that reduce signals associated with neutrophil recruitment and reducing proteins that induce neutrophil recruitment within an individual's gingival crevice, wherein the proteins that induce neutrophil recruitment are TNFα and MIF and the method comprises applying to the individual's oral cavity in an amount effective to increase proteins that reduce signals associated with neutrophil recruitment and that reduce proteins that induce neutrophil recruitment within an individual's gingival crevice.

2. The oral care composition for use according to claim 1 wherein the oral care composition is a toothpaste; or wherein the zinc phosphate is a preformed salt of zinc phosphate.

3. The oral care composition for use according to any of claims 1-2 wherein zinc phosphate is present in an amount sufficient so that the stannous fluoride dissociates to provide a therapeutically effective amount of stannous ions in aqueous solution; and/or wherein the amount of zinc phosphate is from 0.05 to 5% by weight, relative to the weight of the oral care composition.

4. The oral care composition for use according to any of claims 1-3 wherein the amount of the stannous fluoride is from 0.05% to 5% by weight relative to the weight of the oral care composition.

5. The oral care composition for use according to any of claims 1-4 wherein the amount of the water is about 10% by weight or more, relative to the weight of the oral care composition; and/or wherein the amount of the water is about 12% by weight or more, relative to the weight of the oral care composition.

6. The oral care composition for use according to any of claims 1-5, wherein the organic buffer system comprises a carboxylic acid and one or more conjugate base salts thereof, for example, alkali metal salts thereof; preferably wherein the acid is selected from citric acid, lactic acid, malic acid, maleic acid, fumaric acid, acetic acid, succinic acid, and tartaric acid.

7. The oral care composition for use according to claim 6, wherein the one or more conjugate base salts are independently selected from sodium and potassium salts, or combinations thereof.

8. The oral care composition for use according to any of claims 6-7, wherein the acid is citric acid, and the one or more conjugate base salts comprise monosodium citrate (monobasic), di sodium citrate (dibasic), trisodium citrate (tribasic), and combinations thereof.

9. The oral care composition for use according to any of claims 1-8, wherein the composition comprises the organic acid buffer system in an amount of 0. 1 to 5.0% by weight of the composition, measured as the combined amount of organic acid and any conjugate base salt.

10. The oral care composition for use according to any of claims 1-9, wherein the buffer system comprises citric acid and a sodium citrate salt, in a ratio of from 1:3 to 1:6.

11. The oral care composition for use according to any of claims 1-10, wherein the oral care composition further comprises one or more of: an abrasive, one or more humectants, one or more surfactants. one or more alkali phosphate salts, and a whitening agent.

12. The oral care composition for use according to any of claims 1-11, wherein the oral care composition further comprises one or more sources of zinc ions in addition to the zinc phosphate.

13. The oral care composition for use according to any of claims 1-12, wherein the oral care composition is a dentifrice, powder, cream, strip, gum or gel.

14. The oral care composition for use according to any of claims 1-13, wherein the oral care composition comprises:
from 0.5 to 3% by weight zinc phosphate;
from 0.05 to 1 1% by weight stannous fluoride;
from 1 to 8% by weight alkali phosphate salts selected from sodium phosphate dibasic, potassium phosphate dibasic, dicalcium phosphate dihydrate, tetrasodium pyrophosphate, tetrapotassium pyrophosphate, calcium pyrophosphate, sodium tripolyphosphate, and mixtures of any two or more of these, relative to the weight of the oral care composition; and
a silica abrasive.

15. The oral care composition for use according to any of claims 1-14, wherein the pH of the oral care composition is less than 7.

## Patentansprüche

1. Mundpflegezusammensetzung umfassend: Zinkphosphat, Zinn(II)-fluorid und gegebenenfalls ein organisches Säurepuffersystem zur Verwendung in einem Verfahren zum Erhöhen von Proteinen, die mit der Neutrophilenrekrutierung assoziierte Signale verringern, und Reduzieren von Proteinen, die die Neutrophilenrekrutierung in der Zahnfleischspalte eines Individuums induzieren, wobei die Proteine, die die Neutrophilenrekrutierung induzieren, TNFα und MIF sind und das Verfahren das Auftragen in die Mundhöhle des Individuums in einer Menge umfasst, die wirksam ist, um Proteine zu erhöhen, die mit der Neutrophilenrekrutierung assoziierte Signale reduzieren, und die Proteine zu reduzieren, die die Neutrophilenrekrutierung innerhalb der Zahnfleischspalte eines Individuums induzieren.

2. Mundpflegezusammensetzung zur Verwendung gemäß Anspruch 1, wobei die Mundpflegezusammensetzung eine Zahnpasta ist; oder wobei das Zinkphosphat ein vorgeformtes Salz von Zinkphosphat ist.

3. Mundpflegezusammensetzung zur Verwendung nach einem beliebigen der Ansprüche 1-2, wobei Zinkphosphat in einer Menge vorhanden ist, die ausreichend ist, sodass das Zinn(II)-fluorid dissoziiert, um eine therapeutisch wirksame Menge an Zinn(II)-ionen in wässriger Lösung bereitzustellen; und/oder wobei die Menge an Zinkphosphat 0,05 bis 5 Gewichts-%, bezogen auf das Gewicht der Mundpflegezusammensetzung, beträgt.

4. Mundpflegezusammensetzung zur Verwendung nach einem beliebigen der Ansprüche 1-3, wobei die Menge des Zinn(II)-fluorids 0,05 bis 5 Gewichts-%, bezogen auf das Gewicht der Mundpflegezusammensetzung, beträgt.

5. Mundpflegezusammensetzung zur Verwendung nach einem beliebigen der Ansprüche 1-4, wobei die Menge des Wassers etwa 10 Gewichts-% oder mehr, bezogen auf das Gewicht der Mundpflegezusammensetzung, beträgt; und/oder wobei die Menge des Wassers etwa 12 Gewichts-% oder mehr, bezogen auf das Gewicht der Mundpflegezusammensetzung, beträgt.

6. Mundpflegezusammensetzung zur Verwendung nach einem beliebigen der Ansprüche 1-5, wobei das organische Puffersystem eine Carbonsäure und ein oder mehrere konjugierte Basensalze davon, beispielsweise Alkalimetallsalze davon, umfasst, wobei die Säure vorzugsweise ausgewählt ist aus Zitronensäure, Milchsäure, Äpfelsäure, Maleinsäure, Fumarsäure, Essigsäure, Bernsteinsäure und Weinsäure.

7. Mundpflegezusammensetzung zur Verwendung nach Anspruch 6, wobei das eine oder die mehreren konjugierten Basensalze unabhängig voneinander aus Natrium- und Kaliumsalzen oder Kombinationen davon ausgewählt sind.

8. Mundpflegezusammensetzung zur Verwendung nach einem beliebigen der Ansprüche 6-7, wobei die Säure Zitronensäure ist und das eine oder die mehreren konjugierten Basensalze Mononatriumcitrat (monobasisch), Dinatriumcitrat (dibasisch), Trinatriumcitrat (tribasisch) und Kombinationen davon umfassen.

9. Mundpflegezusammensetzung zur Verwendung nach einem beliebigen der Ansprüche 1-8, wobei die Zusammensetzung das organische Säurepuffersystem in einer Menge von 0,1 bis 5,0 %, bezogen auf das Gewicht der Zusammensetzung, gemessen als die kombinierte Menge der organischen Säure und jedes konjugierten Basensalzes, umfasst.

10. Mundpflegezusammensetzung zur Verwendung nach einem beliebigen der Ansprüche 1-9, wobei das Puffersystem Zitronensäure und ein Natriumcitratsalz in einem Verhältnis von 1:3 bis 1:6 umfasst.

11. Mundpflegezusammensetzung zur Verwendung nach einem beliebigen der Ansprüche 1-10, wobei die Mundpflegezusammensetzung weiterhin eines oder mehrere der folgenden umfasst: ein Abrasiv, ein oder mehrere Feuchthaltemittel, ein oder mehrere Tenside, ein oder mehrere Alkaliphosphatsalze und ein Aufhellungsmittel.

12. Mundpflegezusammensetzung zur Verwendung nach einem beliebigen der Ansprüche 1-11, wobei die Mundpflegezusammensetzung weiterhin eine oder mehrere Quellen von Zinkionen zusätzlich zu dem Zinkphosphat umfasst.

13. Mundpflegezusammensetzung zur Verwendung nach einem beliebigen der Ansprüche 1-12, wobei die Mundpflegezusammensetzung ein Zahnputzmittel, Pulver, Creme, Streifen, Kaugummi oder Gel ist.

14. Mundpflegezusammensetzung zur Verwendung nach einem beliebigen der Ansprüche 1-13, wobei die Mundpflegezusammensetzung umfasst:
von 0,5 bis 3 Gewichts-% Zinkphosphat;
von 0,05 bis 11 Gewichts-% Zinn(II)-fluorid;
von 1 bis 8 Gewichts-% Alkaliphosphatsalze, ausgewählt aus zweibasigem Natriumphosphat, zweibasigem Kaliumphosphat, Dicalciumphosphatdihydrat, Tetranatriumpyrophosphat, Tetrakaliumpyrophosphat, Calciumpyrophosphat, Natriumtripolyphosphat und Mischungen von zwei oder mehreren dieser Salze, bezogen auf das Gewicht der Mundpflegezusammensetzung; und
ein Siliziumdioxidabrasiv.

15. Mundpflegezusammensetzung zur Verwendung nach einem beliebigen der Ansprüche 1-14, wobei der pH-Wert der Mundpflegezusammensetzung weniger als 7 beträgt.

## Revendications

1. Composition de soins bucco-dentaires comprenant : du phosphate de zinc, du fluorure stanneux et éventuellement un système tampon d'acide organique pour une utilisation dans un procédé d'augmentation des protéines qui réduisent les signaux associés au recrutement des neutrophiles et de réduction des protéines qui induisent le recrutement des neutrophiles dans le sillon gingival d'un individu, dans laquelle les protéines qui induisent le recrutement des neutrophiles sont le TNFα et le MIF et le procédé comprend l'application à la cavité buccale de l'individu en une quantité efficace pour augmenter les protéines qui réduisent les signaux associés au recrutement des neutrophiles et qui réduisent les protéines qui induisent le recrutement des neutrophiles dans le sillon gingival d'un individu.

2. Composition de soins bucco-dentaires pour une utilisation selon la revendication 1, dans laquelle la composition de soins bucco-dentaires est un dentifrice ; ou dans laquelle le phosphate de zinc est un sel préformé de phosphate de zinc.

3. Composition de soins bucco-dentaires pour une utilisation selon l'une quelconque des revendications 1 à 2, dans laquelle le phosphate de zinc est présent en une quantité suffisante pour que le fluorure stanneux se dissocie pour fournir une quantité thérapeutiquement efficace d'ions stanneux en solution aqueuse ; et/ou dans laquelle la quantité de phosphate de zinc est de 0,05 à 5 % en poids, par rapport au poids de la composition de soins bucco-dentaires.

4. Composition de soins bucco-dentaires pour une utilisation selon l'une quelconque des revendications 1 à 3, dans laquelle la quantité de fluorure stanneux est de 0,05 % à 5 % en poids par rapport au poids de la composition de soins bucco-dentaires.

5. Composition de soins bucco-dentaires pour une utilisation selon l'une quelconque des revendications 1 à 4, dans laquelle la quantité d'eau est d'environ 10 % en poids ou plus, par rapport au poids de la composition de soins bucco-dentaires ; et/ou dans laquelle la quantité d'eau est d'environ 12 % en poids ou plus, par rapport au poids de la composition de soins bucco-dentaires.

6. Composition de soins bucco-dentaires pour une utilisation selon l'une quelconque des revendications 1 à 5, dans laquelle le système tampon organique comprend un acide carboxylique et un ou plusieurs sels de base conjugués de ce dernier, par exemple des sels de métal alcalin de ce dernier ; de préférence dans laquelle l'acide est choisi parmi l'acide citrique, l'acide lactique, l'acide malique, l'acide ma-léique, l'acide fumarique, l'acide acétique, l'acide succinique et l'acide tartrique.

7. Composition de soins bucco-dentaires pour une utilisation selon la revendication 6, dans laquelle lesdits un ou plusieurs sels de base conjugués sont indépendamment choisis parmi les sels de sodium et de potassium, ou les combinaisons de ceux-ci.

8. Composition de soins bucco-dentaires pour une utilisation selon l'une quelconque des revendications 6 à 7, dans laquelle l'acide est l'acide citrique, et lesdits un ou plusieurs sels de base conjugués comprennent le citrate monosodique (monobasique), le citrate disodique (dibasique), le citrate trisodique (tribasique) et les combinaisons de ceux-ci.

9. Composition de soins bucco-dentaires pour une utilisation selon l'une quelconque des revendications 1 à 8, dans laquelle la composition comprend le système tampon d'acide organique en une quantité de 0,1 à 5,0 % en poids de la composition, mesurée en tant que quantité combinée d'acide organique et de tout sel de base conjugué.

10. Composition de soins bucco-dentaires pour une utilisation selon l'une quelconque des revendications 1 à 9, dans laquelle le système tampon comprend de l'acide citrique et un sel de citrate de sodium, en un rapport de 1:3 à 1:6.

11. Composition de soins bucco-dentaires pour une utilisation selon l'une quelconque des revendications 1 à 10, dans laquelle la composition de soins bucco-dentaires comprend en outre un ou plusieurs éléments parmi : un abrasif, un ou plusieurs humectants, un ou plusieurs tensioactifs, un ou plusieurs sels de phosphate alcalin et un agent de blanchiment.

12. Composition de soins bucco-dentaires pour une utilisation selon l'une quelconque des revendications 1 à 11, dans laquelle la composition de soins bucco-dentaires comprend en outre une ou plusieurs sources d'ions zinc en plus du phosphate de zinc.

13. Composition de soins bucco-dentaires pour une utilisation selon l'une quelconque des revendications 1 à 12, dans laquelle la composition de soins bucco-dentaires est un dentifrice, une poudre, une crème, une bandelette, une gomme ou un gel.

14. Composition de soins bucco-dentaires pour une utilisation selon l'une quelconque des revendications 1 à 13, dans laquelle la composition de soins bucco-dentaires comprend :
de 0,5 à 3 % en poids de phosphate de zinc ;
de 0,05 à 11 % en poids de fluorure stanneux ;
de 1 à 8 % en poids de sels de phosphate alcalin choisis parmi le phosphate de sodium dibasique, le phosphate de potassium dibasique, le phosphate dicalcique dihydraté, le pyrophosphate tétrasodique, le pyrophosphate tétrapotassique, le pyrophosphate de calcium, le tripolyphosphate de sodium et les mélanges de deux ou plus de ceux-ci, par rapport au poids de la composition de soins bucco-dentaires ; et
un abrasif à base de silice.

15. Composition de soins bucco-dentaires pour une utilisation selon l'une quelconque des revendications 1 à 14, dans laquelle le pH de la composition de soins bucco-dentaires est inférieur à 7.
